(19) Europäisches Patentamt
European Patent Office
Office européen des brevets

(11) Numéro de publication : 0 296 048 B1

# (12) FASCICULE DE BREVET EUROPEEN

(45) Date de publication du fascicule du brevet : **21.11.91 Bulletin 91/47**

(51) Int. Cl.[5] : **C07D 471/04**, C07D 471/14, C07D 401/12, C07D 403/12, A61K 31/495, A61K 31/50, A61K 31/505

(21) Numéro de dépôt : **88401458.0**

(22) Date de dépôt : **14.06.88**

## (54) Nouveaux dérivés de la pipérazinyl alkyl piperazine dione, leurs procédés de préparation et les compositions pharmaceutiques qui les contiennent.

(30) Priorité : **15.06.87 FR 8708263**

(43) Date de publication de la demande : **21.12.88 Bulletin 88/51**

(45) Mention de la délivrance du brevet : **21.11.91 Bulletin 91/47**

(84) Etats contractants désignés : **AT BE CH DE FR GB IT LI LU NL SE**

(56) Documents cités : **FR-A- 2 013 813**

(73) Titulaire : **ADIR ET COMPAGNIE**
**1 rue Carle Hébert**
**F-92415 Courbevoie Cédex (FR)**

(72) Inventeur : **Lavielle, Gilbert**
**1 Avenue Lilly**
**F-78170 La Celle Saint-Cloud (FR)**
Inventeur : **Poignant, Jean Claude**
**La Guyonnerie 13 Rue de la Fontaine**
**St-Mathieu**
**F-91440 Bures Sur Yvette (FR)**

EP 0 296 048 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés polycycliques azotés, dérivés de la pipérazinyl alkyl pipérazine dione-2,6, leurs procédés de préparation et les compositions pharmaceutiques qui les contiennent.

On connait certains dérivés de la pipérazine dione ayant des effets sédatifs sur le système nerveux central. (De Jong D. et coll. J. Pharm., (1959), 11, p.393-399). Certaines phénylpipérazines ayant une activité anticoagulante sont aussi décrites dans littérature (Demande de Brevet FR 2.013.813).

Les nouveaux dérivés de la pipérazine dione possèdent des propriétés pharmacologiques très intéressantes et notamment des propriétés anxiolytiques antiagressives et antipsychotiques très puissantes. En revanche, ils sont dépourvus des effets secondaires classiquement mecontrés dans cette classe pharmacologique et plus spécialement des effets anticonvulsivants. Ils se distinguent donc par ce fait d'autres dérivés de la pipérazine dione déjà connus. D'autre part, les composés de l'invention par leur structure chimique se démarquent nettement des autres pipérazines et de leurs dérivés déjà décrits.

La présente invention a plus particulièrement pour objet les dérivés de la pipérazinyl alkyl pipérazine dione de formule générale I,

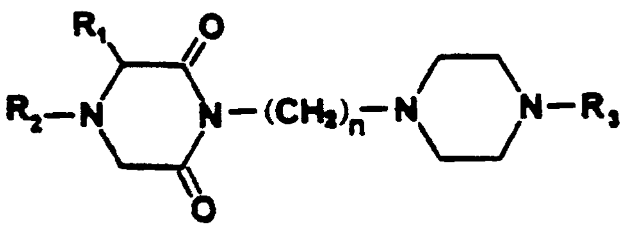

(I)

dans laquelle,

— soit

— $R_1$ représente un atome d'hydrogène,

et

— $R_2$ représente un radical benzyle éventuellement substitué sur le cycle benzénique par un atome d'halogène ou par un radical alcoxyle renfermant de 1 à 4 atomes de carbone,

— soit

— $R_1$ et $R_2$ forment ensemble et avec le radical pipérazine dione-2,6 auquel ils sont attachés un radical hexahydro pyrazino isoquinoléine dione ou un radical hexahydro pyrazino β-carboline dione,

— $R_3$ représente un radical quinolyle, éventuellement substitué par un radical phényle ou par un radical nitro, un radical indolyle substitué à l'atome d'azote par un radical acyle renfermant de 1 à 5 atomes de carbone, ou, à condition toutefois que $R_1$ ne représente pas simultanément un atome d'hydrogène, un radical pyrimidinyle, ou un radical phényle éventuellement substitué par un radical alkyle de 1 à 4 atomes de carbone ou un radical trifluorométhyle,

— n est un nombre entier pouvant prendre les valeurs de 2 à 4,

et leurs sels d'addition à un acide minéral ou organique, pharmaceutiquement acceptable.

La présente invention a également pour objet le procédé de préparation de composé de formule générale I, caractérisé en ce que :

— l'on fait réagir une imide de formule générale II :

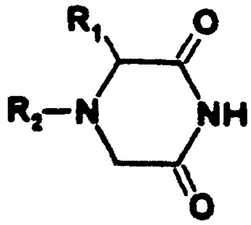

(II)

dans laquelle $R_1$ et $R_2$ ont la signification précédemment définie pour la formule I, sur un alkyle dihalogéné de formule générale III :

$Br-(CH_2)_n-Cl$ (III)

dans laquelle n est un nombre entier pouvant prendre les valeurs de 2 à 4, en présence d'un hydrure de métal alcalin, pour obtenir le composé de formule générale IV :

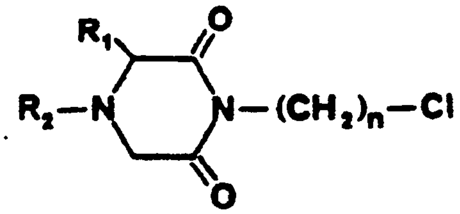

(IV)

dans laquelle $R_1$, $R_2$ et n ont la signification précédemment définie, lequel ensuite l'on condense

— soit avec une pipérazine de formule générale V :

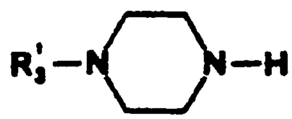

(V)

dans laquelle $R'_3$ représente un radical pyrimidinyle, un radical phényle éventuellement substitué par un radical alkyle de 1 à 4 atomes de carbone ou un radical trifluorométhyle, ou un radical quinolyle éventuellement substitué par un radical phényle ou par un groupement nitro,
afin d'obtenir un composé de formule générale I dans laquelle $R_3$ a la même signification que $R'_3$

— soit avec la benzyloxycarbonyl-4 pipérazine de formule générale VI :

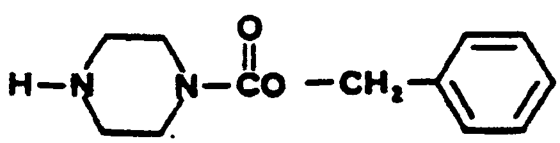

(VI)

pour obtenir un composé de formule générale VII :

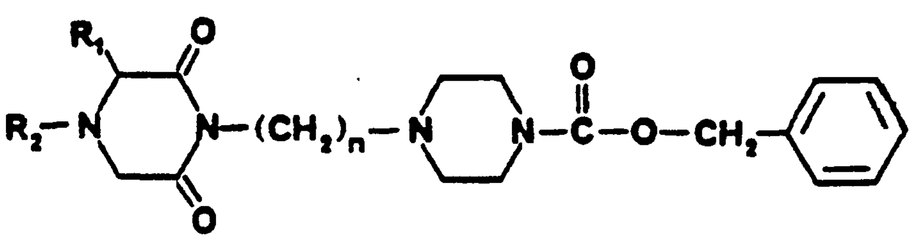

(VII)

dans laquelle $R_1$, $R_2$ et n ont la signification définie ci-dessus,
lequel ensuite l'on soumet à une acidolyse pour obtenir un dérivé de pipérazine de formule VIII,

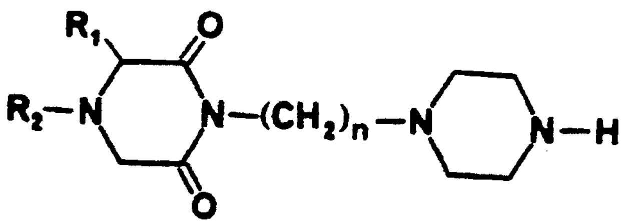

(VIII)

dans laquelle la signification de $R_1$, $R_2$ et n reste identique à celle donnée précédemment, que l'on condense avec un dérivé indolique de formule générale IX :

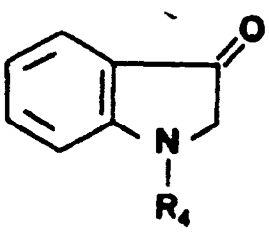

(IX)

dans laquelle $R_4$ représente un radical acyle renfermant de 1 à 5 atomes de carbone, pour former les composés de formule générale I dans laquelle $R_3$ représente un radical indolyle substitué à l'atome d'azote par un radical acyle renfermant de 1 à 5 atomes de carbone, et ensuite, si l'on désire : l'on transforme les composés de la formule générale I en un sel d'addition avec un acide organique ou minéral pharmaceutiquement acceptable.

Les imides de formule générale II sont obtenues par chauffage des diacides adéquats en présence de formamide. Ces derniers sont préparés par l'action de l'acide chloro-2 acétique sur les amines primaires correspondantes (Organic Synthèse Collective ; John Wiley and Sons Ed., N.Y., (1943), vol. II, p. 397). Elles peuvent être aussi synthétisées par alkylation de la pipérazine dione-2,6 par un halogénure d'alcane, (Bull. Soc. Chim. France, (1968), 88, p. 3248).

La condensation des pipérazine diones-2,6 de formule générale IV avec les pipérazines de formule générale V est réalisée dans un solvant organique polaire tel que la butanone-2 en présence de sels minéraux tels que le carbonate de sodium et l'iodure de sodium à une température comprise entre 40°C et 100°C.

L'acidolyse des pipérazine diones-2,6 de formule VII est effectuée par un mélange d'acide acétique et d'acide bromhydrique 72/25, à température ambiante, dans un solvant organique inerte comme l'éther éthylique.

La condensation des pipérazine diones-2,6, de formule générale VIII avec les dérivés indoliques N-acylés de formule générale IX est réalisée dans le toluène anhydre en présence d'un catalyseur acide, comme par exemple, l'acide para-toluènesulfonique.

Parmi les acides pharmaceutiquement acceptables, pour la préparation des sels d'addition aux composés de formule générale I, on peut citer, les acides phosphorique, chlorhydrique, citrique, oxalique, sulfurique, tartrique, mandélique, triméthanesulfonique, etc...

Les composés selon l'invention, ainsi que leurs sels dont doués de propriétés pharmacologiques fort intéressantes.

En effet, les essais pharmacologiques in vivo ont montré que ces composés possèdent de puissantes propriétés anxiolytiques, antiagressives et antipsychotiques. Ces propriétés ont été mises en évidence au moyen d'essais classiquement utilisés chez l'animal permettant de présager avec une très bonne précision l'activité anxiolytique, antiagressive ou antipsychotique des nouveaux composés chez l'homme. (Dallas Treit., Neur. Biob. Rev., (1985), 9, p. 203-222).

En revanche, les composés de la présente invention sont dépourvus de propriétés sédatives du système nerveux central et se distinguent d'autres dérivés de la pipérazine dione-2,6 déjà connus.

Les composés de la présente invention présentent en particulier un profil d'agent psychotrope anxiosélectif. Leurs propriétés pharmacologiques permettent leur application dans le traitement de l'anxiété sous toutes ses formes.

L'invention s'étend aussi aux compositions pharmaceutiques renfermant comme principe actif au moins un composé de formule générale I ou l'un de ses sels d'addition avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie d'administration.

La voie d'administration préférée est la voie orale ou parentérale. D'une manière générale, la posologie unitaire s'échelonnera entre 0,1 et 100 mg et la posologie journalière, utilisable en thérapeutique humaine entre 0,1 et 300 mg.

Les exemples suivants, donnés à titre non-limitatif, illustrent l'invention.

Les points de fusion indiqués sont mesurés selon la technique micro-Köfler. Les spectres infra-rouge sont obtenus avec des solutions des produits dans le Nujol. Les spectres de résonance magnétique nucléaire du proton (R.M.N.) ont été enregistrés à 60 MHz.

## EXEMPLE 1

**Dichlorhydrate de la {[(trifluorométhyl-3 phényl)-4 pipérazinyl-1]-3 propyl}-2 hexahydro-1,3,4,6,11,11a-2H-pyrazino [1,2-b] isoquinoléine dione-1,3**

### Stade A

• Acide carboxy-3 tétrahydro-1,2,3,4 isoquinolyl-2 acétique

A 25°C, ajouter 2,25 moles d'hydroxyde de sodium en solution dans 280 ml d'eau, dans une solution de 1,12 moles de chloro-2 acétate de sodium dans 500 ml d'eau. Introduire ensuite, 1,12 moles d'acide tétrahydro-1,2,3,4 isoquinoléique-2. Porter le milieu réactionnel à environ 70°C, jusqu'à l'obtention d'une solution limpide.

Ajouter, à 40°C, une solution de 1,18 moles de chlorure de baryum dans 600 ml d'eau. Porter ensuite à 90°C, pendant 15 minutes, puis filtrer rapidement. Rincer le précipité par deux fois 350 ml d'eau chaude. Sécher et placer 0,85 mole de ce précipité dans 800 ml d'eau. Ajouter 0,92 mole d'acide sulfurique en solution dans l'eau. Porter 10 minutes à reflux. Filtrer le précipité de sulfate de baryum formé. Concentrer la phase aqueuse jusqu'à précipitation du diacide. Filtrer. Sécher.

Point de fusion : 218°C
Rendement : 65%

### Stade B

• Hexahydro-1,3,4,6,11,11a-2H-pyrazino [1,2-b] isoquinoléine dione-1,3

Chauffer pendant 1 heure et demie à 120°C puis 5 heures à 160°C une suspension de 0,46 mole du diacide précédemment obtenu, dans 300 ml de formamide. Refroidir ensuite à 20°C pour obtenir la cristallisation de l'imide. Filtrer. Rincer à l'éther.

Point de fusion : 182°C
Rendement : 60%

### Stade C

• (chloro-3 propyl)-2 hexahydro-1,3,4,6,11,11a-2H-pyrazino [1,2-b] isoquinoléine dione-1,3

A une suspension de 0,046 mole d'hydrure de sodium dans 100 ml de diméthylformamide, ajouter une solution de 0,046 mole de l'imide précédemment obtenue dans 60 ml de diméthylformamide. Porter à 60°C pendant 45 minutes. Refroidir à 25°C et ajouter 0,05 mole de bromo-1 chloro-3 propane. Maintenir l'agitation à 25°C jusqu'à la disparition de l'imide. Evaporer le solvant sous vide. Reprendre le résidu au benzène. Laver à l'eau. Sécher la phase organique sur sulfate de sodium anhydre. Concentrer sous vide. Cristalliser dans un mélange éther-acétone.

Point de fusion : 97°C
Rendement : 72%

Stade D

Porter à reflux 48 heures un mélange de 0,03 mole du composé obtenu ci-dessus, 0,03 mole de (trifluorométhyl-3 phényl)-4 pipérazine, 5 g de carbonate de sodium, et 0,5 g d'iodure de sodium dans 200 ml de butanone-2. Refroidir, filtrer les sels minéraux, concentrer sous vide. Reprendre le résidu dans l'eau et extraire au benzène. Sécher la phase organique sur sulfate de sodium anhydre. Evaporer le solvant sous vide. Cristalliser dans un mélange d'éther isopropylique et d'éther de pétrole (10 : 90 v/v). Recristalliser dans l'éther isopropylique.

Dissoudre le produit obtenu dans le minimum d'acétone. Ajouter deux équivalents d'éthanol chlorhydrique. Filtrer le dichlorhydrate obtenu.

Point de fusion : 167°C
Rendement : 65%

Les constantes physiques spectrales de la base sont indiquées dans le Tableau I.

EXEMPLE 2

**Tartrate de la {[(pyrimidyl-2)-4 pipérazinyl-1]-4 butyl}-2 hexahydro-1,3,4,6,11,11a-2H-pyrazino [1,2-b] isoquinoléine dione-1,3**

Stade A

**(chloro-4 butyl)-2 hexahydro-1,3,4,6,11,11a-2H-pyrazino [1,2-b] isoquinoléine dione-1,3**

Ce composé a été préparé selon le procédé décrit dans le stade C de l'exemple 1 et en utilisant le bromo-1 chloro-4 butane au lieu de bromo-1 chloro-3 propane et en chauffant pendant une heure à environ 90°C. Pour purifier le composé brut chromatographier sur colonne de silice, en utilisant comme solvant d'élution le dichlorométhane et ensuite concentrer l'éluat sous vide.
Rendement : 50%

Stade B

La {[(pyrimidyl-2)-4 pipérazinyl-1]-4 butyl}-2 hexahydro-1,3,4,6,11,11a-2H-pyrazino [1,2-b] isoquinoléine dione-1,3 est obtenue selon le procédé décrit dans l'exemple 1, Stade D, et en utilisant la (pyrimidinyl-2)-4 pipérazine au lieu de la (trifluorométhyl-3 phényl)-4 pipérazine.

Elle a été purifiée sur colonne de silice en utilisant comme éluant un mélange de dichlorométhane éthanol (gradient 99 : 1 à 95 : 5 v/v). Les constantes physiques spectrales de cette base sont indiquées dans le Tableau I.

Après concentration sous vide, le résidu est solubilisé dans le minimum d'éthanol. Ajouter la quantité stoechiométrique d'acide DL, tartrique nécessaire, en solution dans l'éthanol. Concentrer sous vide, cristalliser dans l'éther éthylique. Filtrer.

Point de fusion : 92°C
Rendement : 60%

EXEMPLE 3

**Dichlorhydrate de la {[(trifluorométhyl-3 phényl)-4 pipérazinyl-1]-3 propyl}-2 hexahydro-1,3,4,6,12,12a-2H-pyrazino [1,2-b] β-carboline dione-1,3**

Ce composé a été préparé selon le procédé décrit dans l'exemple 1. L'acide de départ utilisé au stade A est décrit dans J. Med. Chem., (1973), 16, N°4, p. 419.

Rendement : 40%

Point de fusion : 195°C

Les constantes physiques spectrales de la {[(trifluorométhyl-3 phényl)-4 pipérazinyl-1]-3 propyl}-2 hexahydro-1,3,4,6,12,12a pyrazino [1,2-b] β-carboline dione-1,3 sont données au Tableau I.

**EXEMPLE 4**

**{[(pyrimidyl-2)-4 pipérazinyl-1]-4 butyl}-2 hexahydro-1,3,4,6,12,12a-2H-pyrazino [1,2-b] β-carboline dione-1.3.**

Ce composé a été préparé selon le procédé décrit dans l'exemple 1, mais en utilisant au stade A, l'acide adéquat, au Stade C le bromo-1 chloro-4 butane et au stade D la (pyrimidinyl-2)-4 pipérazine.

Les constantes physiques spectrales sont indiquées dans le Tableau I.

Point de Fusion : 217°C
Rendement : 45%

**EXEMPLE 5**

**(fluoro-2 benzyl)-4 {[(nitro-6 quinolyl-2)-4 pipérazinyl-1]-3 propyl}-1 pipérazine dione-2,6**

Stade A

• **(chloro-3 propyl)-1 (fluoro-2 benzyl)-4 pipérazine dione-2,6**

Préparer une suspension de 0,12 mole d'hydrure de sodium dans 100 ml de diméthylformamide. Ajouter 0,12 mole de (fluoro-2 benzyl)-4 pipérazine dione-2,6 en solution dans 50 ml de diméthylformamide. Chauffer à 70°C pendant 30 minutes. Refroidir et ajouter 0,13 mole de bromo-1 chloro-3 propane. Laisser réagir à la température ambiante jusqu'à disparition complète de l'imide. Eliminer ensuite le solvant organique sous vide et reprendre le résidu par 100 ml d'eau et 200 ml de benzène.

Décanter, éliminer la phase aqueuse et évaporer la phase organique. Le résidu est trituré dans l'éther. On obtient des cristaux purs de la (chloro-3 propyl)-1 (fluoro-2 benzyl)-4 pipérazine dione-2,6.
Rendement : 96%.

Stade B

Ajouter dans une solution de 0,025 mole de (chloro-3 propyl)-1 (fluoro-2 benzyl)-4 pipérazine dione-2,6 précédemment obtenue et de 0,0275 mole de (nitro-6 quinolyl-2)-4 pipérazine (J. Chem. Soc., (1949), 54, p. 227-229 et J. Med. Chem., (1985) 28, p. 1394-98) dans 200 ml de butanone-2, un mélange contenant 6 g de carbonate de sodium et 0,5 g d'iodure de sodium. Porter à reflux pendant 40 heures et ensuite filtrer et concentrer sous vide. Reprendre le résidu dans du benzène et laver la phase benzénique trois fois à l'eau distillée. Sécher la phase organique sur sulfate de sodium anhydre et évaporer le solvant. Purifier le résidu obtenu par chromatographie sur colonne de silice en utilisant comme éluant un mélange de dichlorométhane et de méthanol (95 : 5 v/v).

Point de fusion : 153°C
Rendement : 60%

Les constantes physiques spectrales de cette base sont décrites dans le Tableau I.

**EXEMPLE 6**

**Ditartrate de la (fluoro-2 benzyl)-4 {[(phényl-4 quinolyl-2)-4 pipérazinyl-1]-3 propyl}-1 pipérazine dione-2,6**

Ce composé a été préparé selon le procédé décrit dans l'exemple 5 mais en utilisant au Stade B au lieu de la (nitro-6 quinolyl-2)-4 pipérazine, la (phényl-4 quinolyl-2)-4 pipérazine. La préparation de ce dernier composé est connue. (J. Am. Chem. Soc., (1948), 70, p. 2402 et J. Med. Chem., (1985), 28, p. 1394-98).

Point de fusion : = 95°C
Rendement : 30%

Les constantes physiques spectrales de la base sont données dans le Tableau I.

**EXEMPLE 7**

**Dichlorohydrate de la {[(acétyl-1 indolyl-3)-1 pipérazinyl-4]-3 propyl}-1 (méthoxy-2 benzyl)-4 pipérazine dione-2,6**

Stade A

[(benzyloxycarbonyl-1 pipérazinyl-4)-3 propyl]-1 (méthoxy-2 benzyl)-4 pipérazine dione-2,6.

Ce composé a été préparé selon le procédé décrit dans l'exemple 5 en condensant la (méthoxy-2 benzyl)-4 (chloro-3 propyl)-1 pipérazine dione-2,6 avec la benzyloxycarbonyl-4 pipérazine.

Point de fusion : < 50°C
Rendement : 82%

La benzyloxycarbonyl-4 pipérazine est obtenue selon le procédé décrit dans Synthesis,(1984), 12, p. 1032.

Stade B

[(pipérazinyl-4)-3 propyl]-1 (méthoxy-2 benzyl) pipérazine dione-2,6

L'amine obtenue ci-dessus (0,08 mole) est mise en solution dans 200 ml d'un mélange d'acide acétique et d'acide bromhydrique gazeux (75 : 25 p/p). Après 2 heures d'agitation à 25°C, diluer avec 800 ml d'éther. Filtrer le précipité. Rincer à l'éther éthylique. Le précipité est repris dans l'eau et lavé au chloroforme. La phase aqueuse est alors alcalinisée (pH = 10), l'amine est extraite au chloroforme. La phase organique est séchée sur sulfate de sodium et concentrée sous vide.
Rendement : 70%.

Stade C

Porter à reflux un mélange de 0,027 mole d'amine obtenue au stade précédent, de 0,023 mole de N-acétyl indolinone-3 (synthèse selon D. NENITZESCU Rev. Roum. Chem., (1967), 12, (2), p. 105-8), et de 0,015 mole d'acide para-toluènesulfonique dans 150 ml de toluène anhydre. Maintenir le reflux jusqu'à l'obtention de la quantité d'eau théorique, soit 0,7 ml : Concentrer sous vide. Reprendre le résidu dans l'eau. Extraire au benzène. Sécher la phase organique sur sulfate de sodium. Evaporer sous vide. Purifier l'huile obtenue par chromatographie sur silice (300 g). Eluer à l'aide d'un gradient d'éther éthylique — acétone 90 : 10 à 70 : 30 (v/v). Concentrer l'éluant sous vide. Dissoudre le résidu dans 100 ml d'un mélange d'éther éthylique — éthanol (96: 4 v/v). Ajouter deux équivalents d'éthanol chlorhydrique. Filtrer le précipité formé.

Point de fusion : 183°C
Rendement : 20%

Les constantes physiques spectrales de la {[(acétyl-1 indolyl-3)-1 pipérazinyl-4]-3 propyl}-1 (méthoxy-2 benzyl)-4 pipérazine dione-2,6 sont données dans le Tableau I.

TABLEAU I

COMPOSE DE FORMULE GENERALE I

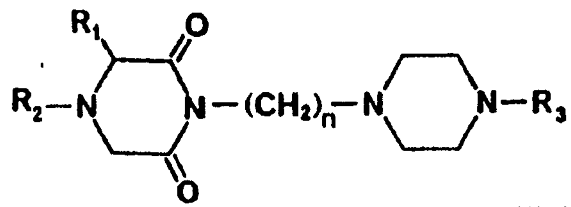

| EX | 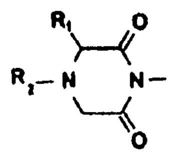 | n | R3 | IR cm-1<br>υ(C=O) | R M N<br>Solvant |
|---|---|---|---|---|---|
| 1 | 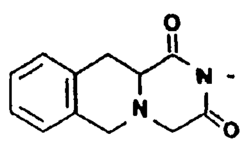 | 3 | 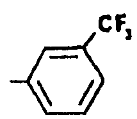 | 1680<br>1730 | ($CDCl_3$)<br>1,8 ppm,m,2H; 2,4 à 3,2 ppm,m,10H; 3 à 4 ppm,m,9H; 6,8 à 7,5 ppm,m,8H |
| 2 | 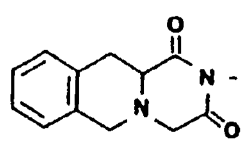 | 4 | 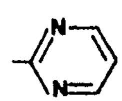 | 1680<br>1730 | ($CDCl_3$)<br>1 à 1,8 ppm,m,4H; 2 à 2,7 ppm,m,6H; 2,7 à 4 ppm,m,13H; 6,4 ppm,t,1H; 7,1 ppm,m,4H; 8,3 ppm,d,2H |
| 3 | 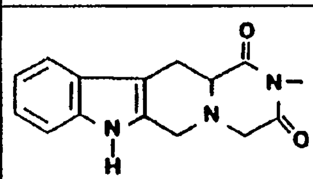 | 3 | 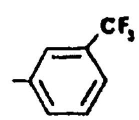 | 1660<br>1730 | ($CDCl_3$)<br>2 ppm,m,2H; 2,3 à 2,9 ppm,m,6H; 2,9 à 3,5 ppm,m,6H; 3,5 à 4,2 ppm,m,7H; 7 à 7,8 ppm,m,8H; 9,6 ppm 1H échangeable |

TABLEAU I (SUITE)

COMPOSE DE FORMULE GENERALE I

| EX | 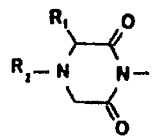 | n | R3 | IR $cm^{-1}$ ν(C=O) | R M N Solvant |
|---|---|---|---|---|---|
| 4 | 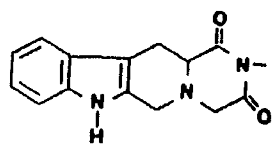 | 4 | 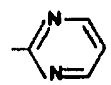 | 1655 1740 | ($CDCl_3$ + DMSO) 1,2 à 1,9 ppm,m,4H; 2,3 à 2,6 ppm,m,6H; 2,9 à 3,3 ppm,m,2H; 3,4 à 3,9 ppm,m,4H; 3,5 à 4 ppm,m,5H; 3,7 à 4,2 ppm,m,2H; 6,5 ppm,m,1H; 6,6 à 7,6 ppm,m,4H; 8,25 ppm,d,2H; 10,6 ppm 1H échangeable |
| 5 | 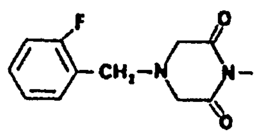 | 3 | 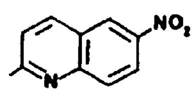 | 1675 1735 | ($CDCl_3$) 1,5 à 2,1 ppm,m,2H; 2,2 à 2,7 ppm,m,6H; 3,4 ppm,s,4H; 3,6 à 4,1 ppm,m,8H; 7 à 7,5 ppm,m,5H; 7,7 ppm,d,1H; 8 ppm,d,1H; 8,4 ppm,dd,1H; 8,6 ppm,d,1H |
| 6 | 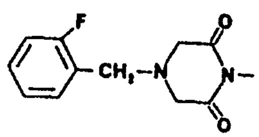 | 3 | 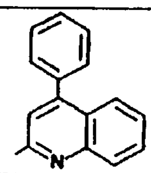 | 1680 1735 | ($CDCl_3$) 1,5 à 2,1 ppm,m,2H; 2,2 à 2,8 ppm,m,6H; 3,4 ppm,s,4H; 3,6 à 4 ppm,m,8H; 6,8 à 8 ppm,m,14H |
| 7 | 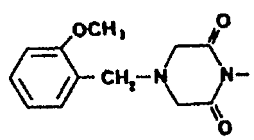 | 3 | 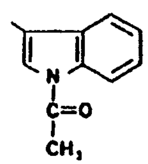 | 1680 1740 | ($CDCl_3$) 1,5 à 1,9 ppm,m,2H; 2,2 ppm,s,3H; 2,3 à 2,9 ppm,m,6H; 3,2 ppm,m,4H; 3,4 ppm,s,4H; 3,8 ppm,s,3H; 3,6 à 4 ppm,t,2H et s,2H; 6,7 à 7,6 ppm,m,9H |

## ETUDE PHARMACOLOGIQUE

## EXEMPLE 8

### Evaluation de l'activité anxiolytique par le test des quatres plaques

L'activité anxiolytique des composés de la formule générale I a été recherchée chez la souris selon la méthode de Aron, Simon, Larousse et Boissier, décrite dans Neuropharmacology, (1971), 10, p. 459-469. Après traitement des animaux au moyen de différentes doses des composés de l'invention administrées par voie intrapéritonéale ou orale, l'augmentation du pourcentage des transitions lors de l'exploration du fond de la cage-test, en présence de chocs électriques, a été évaluée. Les résultats de cette étude sont indiqués dans les Tableaux II et III.

TABLEAU II

| COMPOSE DE L'EXEMPLE | DOSE i.p. ($mg.kg^{-1}$) | % D'AUGMENTATION DES REPONSES (TRANSITIONS) |
|---|---|---|
| 1 | 10 (S) | 47 |
| 1 | 25 (S) | 57 |
| 2 | 10 (b) | 39 |
| 3 | 50 (b) | 16 |
| 4 | 5 (b) | 22 |

(S) = Sel
(b) = base

TABLEAU III

| COMPOSE DE L'EXEMPLE | DOSE p.o. ($mg.kg^{-1}$) | % D'AUGMENTATION DES REPONSES (TRANSITIONS) |
|---|---|---|
| 1 | 5 (b) | 12 |
| 1 | 10 (b) | 33 |

(b) = base

## EXEMPLE 9

### Inhibition de l'agressivité d'isolement chez la souris

Les composés de formule générale I inhibent le comportement d'attaque des souris rendues agressives par isolement selon la méthode décrite par Yen, Stanger et Millman dans Arch. Int. Pharmacodyn., (1959), 123, p. 179-185. L'effet sur l'agressivité est évalué par le pourcentage des animaux devenus non agressifs ("protégés") après traitement. Les résultats de cet essai sont donnés dans le Tableau IV.

TABLEAU IV

| COMPOSE DE L'EXEMPLE | DOSE i.p. (mg.kg-1) (b) | % DE COUPLES RENDUS NON AGRESSIFS |
|---|---|---|
| 1 | 12,5<br>25,0 | 56 (S)<br>89 (S) |
| 2 | 12,5<br>25,0 | 50 (S)<br>100 (S) |
| 3 | 12,5<br>25,0 | 40 (S)<br>90 (S) |
| 4 | 12,5<br>25,0 | 78 (S)<br>88 (S) |

(S) = Significatif $p < 0,05$
(b) = base

## EXEMPLE 10

### Inhibition de l'agressivité du rat isolé et bulbectomisé

Les composés de la présente invention inhibent l'agressivité du rat isolé et bulbectomisé. L'inhibition de l'agressivité a été évaluée selon la méthode décrite par Vergnes et Karli dans C.R. Soc. Biol., (1963), 157, p. 1061. L'effet sur l'agressivité est évalué par le pourcentage des animaux devenus non tueurs après traitement. Les résultats de cet essai sont indiqués dans le Tableau V.

TABLEAU V

| EXEMPLE | DOSE i.p. (sel) (mg.kg-1) | % DES ANIMAUX NON TUEURS |
|---|---|---|
| 1 | 25 | 55 |
| 3 | 12,5 | 36 |
| 7 | 12,5 | 25 |

**EXEMPLE 11**

**Inhibition de la réponse conditionnée d'évitement actif chez la rat**

Les résultats obtenus avec les composés de formule générale I au cours des différentes expérimentations mettant en oeuvre la méthode décrite par Courvoisier et coll. dans Arch. Int. Pharmacodyn., (1953), 92, p. 305-361 et par Janssen et coll. dans Arzneim. Forsch, (1965), 15, p. 104-117 sont rapportés dans le Tableau VI. Les pourcentages d'inhibition de la réponse conditionnée d'évitement obtenus (% IRCE) démontrent que les composés de l'invention possèdent une propriété antipsychotique intéressante.

TABLEAU VII

| EXEMPLE | DOSE i.p. (mg.kg-1) | % I.R.C.E. |
|---|---|---|
| 1 | 20 | 11 |
| 2 | 20 | 32 |
| 3 | 20 | 25 |

**EXEMPLE 12**

**Evaluation de l'activité anxiolytique par le test de Conflit de Mac Millan**

Dans ces expériences de conditionnement opérant en présence d'une récompense alimentaire, effectuées chez le rat, selon la méthode décrite par Mac Millan dans Fed. Proceedings, (1975), 34, (9), p. 1870-1879, on considère deux paramètres dans les réponses comportementales de l'animal. Le premier paramètre est la variation du taux de réponses punies, en présence d'un choc électrique, sous l'influence du traitement par rapport au taux de réponses punies sous l'influence du sérum physiologique. Le deuxième paramètre est la variation du taux de réponses non punies, sans choc électrique, sous l'influence du même traitement, par rapport au taux de réponses non punies, lors de l'administration de sérum physiologique. Un effet anxiolytique spécifique est constaté lorsqu'après l'administration d'une substance le taux des seules réponses punies se trouve augmenté. Comme les résultats indiqués dans le Tableau VII le démontrent, le composé de l'exemple 1 à la dose de 10 mg.kg-1 administré par voie intrapéritonéale, donné des résultats positifs.

TABLEAU VII

| EXEMPLE | % DE VARIATION DES REPONSES PUNIES | % DE VARIATION DES REPONSES NON PUNIES |
|---|---|---|
| 1 | + 49 S | + 10 NS |

S = significatif $p < 0,016$

EXEMPLE 13

Recherche des effets secondaires

L'effet anti-convulsivant des composés de l'invention a été évalué chez la souris en recherchant un antagonisme vis-à-vis des convulsions induites par une dose de 100 $mg \cdot kg^{-1}$ de pentylènetétrazol administrée par voie intrapéritonéale.

Les composés de formule générale I administrés à la dose de 50 $mg \cdot kg^{-1}$ par voie intrapéritonéale ne protègent pas les animaux des convulsions. Ils ne possèdent donc pas, à cette dose, de composante anti-convulsivante importante.

**PREPARATION PHARMACEUTIQUE**

**EXEMPLE 14 :**

**Comprimés dosés à 2 mg de dichlorhydrate de la {[(trifluorométhyl-3 phényl)-4 pipérazinyl-1]-3 propyl}-2 hexahydro-1,2,3,4,11,11a-2H-pyrazino [1,2-b] isoquinoléine dione-1,3.**

Dichlorhydrate de la {[(trifluorométhyl-3 phényl)-4 pipérazinyl-1]-3 propyl}-2 hexahydro-1,2,3,4,11,11a-2H-pyrazino [1,2-b] isoquinoléine dione-1,3 .................... 2 g
Amidon de blé .................... 100 g
Amidon de maïs .................... 20 g
Stéarate de magnésium .................... 15 g
Talc .................... 20 g

pour 1000 comprimés à 2 mg de principe actif.

## Revendications

1. Composés de formule générale I :

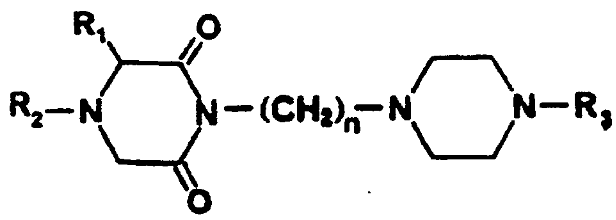

dans laquelle,

— soit

— $R_1$ représente un atome d'hydrogène,

et

— $R_2$ représente un radical benzyle éventuellement substitué sur le cycle benzénique par un atome d'halogène ou par un radical alcoxyle renfermant de 1 à 4 atomes de carbone,

— soit

— $R_1$ et $R_2$ forment ensemble et avec le radical pipérazine dione-2,6 auquel ils sont attachés un radical hexahydro pyrazino isoquinoléine dione ou un radical hexahydro pyrazino β-carboline dione,

— $R_3$ représente un radical quinolyle, éventuellement substitué par un radical phényle ou par un radical nitro, un radical indolyle substitué à l'atome d'azote par un radical acyle renfermant de 1 à 5 atomes de carbone, ou, à condition toutefois que $R_1$ ne représente pas simultanément un atome d'hydrogène, un radical pyrimidinyle, ou un radical phényle éventuellement substitué par un radical alkyle de 1 à 4 atomes de carbone ou un radical trifluorométhyle,

— n est un nombre entier pouvant prendre les valeurs de 2 à 4, et leurs sels formés d'addition à un acide minéral ou organique, pharmaceutiquement acceptable.

2. La (fluoro-2 benzyl)-4 {[(nitro-6 quinolyl-2)-4 pipérazinyl-1]-3 propyl]-1 pipérazine dione-2,6 et ses sels d'addition avec un acide minéral ou organique pharmaceutiquement acceptable.

3. La {[(acétyl-1 indolyl-3)-1 pipérazinyl-4]-3 propyl}-1 (méthoxy-2 benzyl)-4 pipérazine dione-2,6 et ses sels d'addition avec un acide minéral ou organique pharmaceutiquement acceptable.

4. La {[(trifluorométhyl-3 phényl)-4 pipérazinyl-1]-3 propyl}-2 hexahydro-1,3,4,6,11,11a-2H-pyrazino [1,2-b] isoquinoléine dione-1,3 et ses sels d'addition avec un acide minéral ou organique pharmaceutiquement acceptable.

5. La {[(pyrimidinyl-2)-4 pipérazinyl-1]-4 butyl}-2 hexahydro-1,3,4,6,11,11a-2H-pyrazino [1,2-b] isoquinoléine dione-1,3 et ses sels d'addition avec un acide minéral ou organique pharmaceutiquement acceptable.

6. {[(trifluorométhyl-3 phényl)-4 pipérazinyl-1]-3 propyl}-2 hexahydro-1,3,4,6,12,12a-2H-pyrazino [1,2-b] β-carboline dione-1,3 et ses sels d'addition avec un acide minéral ou organique pharmaceutiquement acceptable.

7. Procédé de préparation de composé de formule générale I, caractérisé en ce que :

— l'on fait réagir une imide de formule générale II :

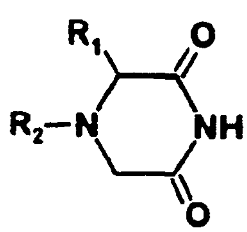

(II)

dans laquelle $R_1$ et $R_2$ ont la signification précédemment définie pour la formule I, sur un alkyle dihalogéné de formule générale III :

$$Br-(CH_2)_n-Cl \qquad (III)$$

dans laquelle n est un nombre entier pouvant prendre les valeurs de 2 à 4, en présence d'un hydrure de métal alcalin, pour obtenir le composé de formule générale IV :

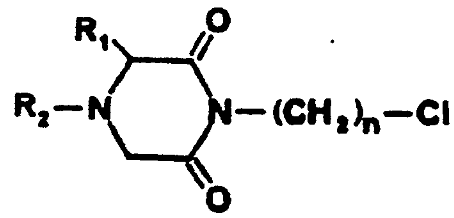

. (IV)

dans laquelle $R_1$, $R_2$ et n ont la signification précédemment définie, lequel ensuite l'on condense

— soit avec une pipérazine de formule générale V :

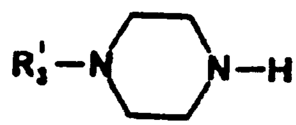

(V)

dans laquelle $R'_3$ représente un radical pyrimidinyle, un radical phényle éventuellement substitué par un radical alkyle de 1 à 4 atomes de carbone ou un radical trifluorométhyle, ou un radical quinolyle éventuellement substitué par un groupement nitro,

afin d'obtenir un composé de formule générale I dans laquelle $R_3$ a la même signification que $R'_3$

— soit avec la benzyloxycarbonyl-4 pipérazine de formule générale VI :

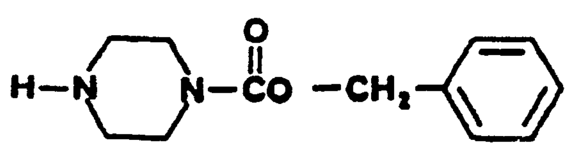

(VI)

pour obtenir un composé de formule générale VII :

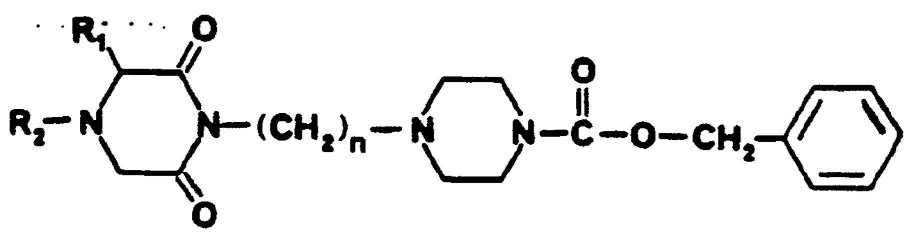

(VII)

dans laquelle $R_1$, $R_2$ et n ont la signification définie ci-dessus,

lequel ensuite l'on soumet à une acidolyse pour obtenir un dérivé de pipérazine de formule VIII,

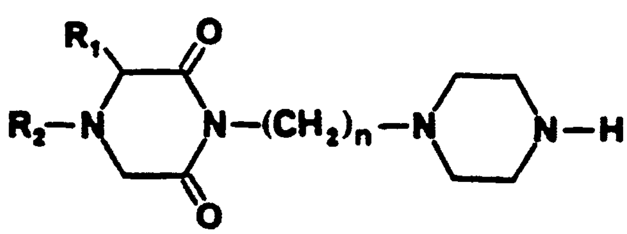

(VIII)

dans laquelle la signification de $R_1$, $R_2$ et n reste identique à celle donnée précédemment,

que l'on condense avec un dérivé indolique de formule générale IX :

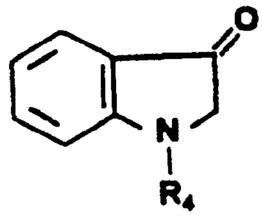

(IX)

dans laquelle $R_4$ représente un radical acyle renfermant de 1 à 5 atomes de carbone,
pour former les composés de formule générale I dans laquelle $R_3$ représente un radical indolyle substitué d'azote par un radical acyle renfermant de 1 à 5 atomes de carbone,
et ensuite, si l'on désire,
l'on transforme les composés de la formule générale I en un sel d'addition avec un acide organique ou minéral pharmaceutiquement acceptable.

8. Composition pharmaceutique contenant comme principe actif un composé selon l'une quelconque des revendications 1 à 6, en association ou en mélange avec un excipient ou un véhicule inerte non toxique, pharmaceutiquement acceptable.

9. Composition pharmaceutique selon la revendication 8 renfermant le principe actif à la dose de 0,1 à 100 mg.

10. Composition pharmaceutique selon les revendications 8 et 9 renfermant comme principe actif au moins un composé selon les revendications 1 à 6 utilisable dans le traitement des maladies nécessitant des médications anxiolytiques, antiagressives, ou antipsychotiques.

**Patentansprüche**

1. Verbindungen der allgemeinen Formel I

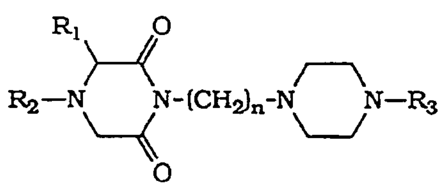

(I)

in der
— entweder
— $R_1$ ein Wasserstoffatom und
— $R_2$ eine Benzylgruppe, die gegebenenfalls am Benzolring durch ein Halogenatom oder eine Alkoxygruppe mit 1 bis 4 Kohlenstoffatomen substituiert ist.
— oder
— $R_1$ und $R_2$ gemeinsam mit dem Piperazin-2,6-dion-Rest, an den sie gebunden sind, eine Hexahydro-pyrazino-isochinolin-dion-Gruppe oder eine Hexahydro-pyrazino-β-carbolin-dion-Gruppe bilden,
— $R_3$ eine Chinolylgruppe, die gegebenenfalls durch eine Phenylgruppe oder eine Nitrogruppe substituiert ist, eine Indolylgruppe, die am Stickstoffatom durch eine Acylgruppe mit 1 bis 5 Kohlenstoffatomen substituiert ist,
oder, mit der Maßgabe, daß $R_1$ nicht gleichzeitig ein Wasserstoffatom darstellt, eine Pyrimidinylgruppe oder eine Phenylgruppe, die gegebenenfalls durch eine Alkylgruppe mit 1 bis 4 Kohlenstoffatomen oder eine Trifluormethylgruppe substituiert ist, und
— n eine ganze Zahl mit einem Wert von 2 bis 4 bedeuten und deren Additionssalze mit einer anorganischen oder organischen, pharmazeutisch annehmbaren Säure.

2. 4-(2-Fluorbenzyl)-1-{3-[4-(6-nitro-chinol-2-yl)-piperazin-1-yl]-propyl}-piperazin-2,6-dion und dessen Additionssalze mit einer anorganischen oder organischen, pharmazeutisch annehmbaren Säure.

3. 1-{3-[1-(1-Acetyl-indol-3-yl)-piperazin-4-yl]-propyl}-4-(2-methoxybenzyl)-piperazin-2,6-dion und dessen Additionssalze mit einer anorganischen oder organischen, pharmazeutisch annehmbaren Säure.

4. 2-{3-[4-(3-Trifluormethyl-phenyl)-piperazin-1-yl]-propyl}-1,3,4,6,11,11a-hexahydro-2H-pyrazino[1,2-b]isochinolin-1,3-dion und dessen Additionssalze mit einer anorganischen oder organischen, pharmazeutisch

annehmbaren Säure.

5. 2-{4-[4-(Pyrimidin-2-yl)-piperazin-1-yl]-butyl}-1,3,4,6,11,11a-hexahydro-2H-pyrazino[1,2-b]isochinolin-1,3-dion und dessen Additionssalze mit einer anorganischen oder organischen, pharmazeutisch annehmbaren Säure.

6. 2-{3-[4-(3-Trifluormethyl-phenyl)-piperazin-1-yl]-propyl}-1,3,4,6,12,12a-hexahydro-2H-pyrazino[1,2-b]β-carbolin-1,3-dion und dessen Additionssalze mit einer anorganischen oder organischen, pharmazeutisch annehmbaren Säure.

7. Verfahren zur Herstellung der Verbindung der allgemeinen Formel I, **dadurch gekennzeichnet**, daß man

— ein Imid der allgemeinen Formel II

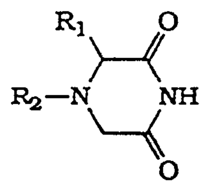

(II)

in der $R_1$und $R_2$ die bezüglich der allgemeinen Fomnel I angegebenen Bedeutungen besitzen, mit einem Dihalogenalkyl der allgemeinen Formel III

$Br-(CH_2)_n-Cl$ (III)

in der n eine ganze Zahl mit Werten von 2 bis 4 bedeutet, in Gegenwart eines Alkalimetallhydrids zu einer Verbindung der allgemeinen Formel IV

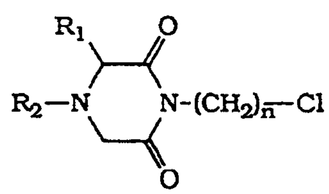

(IV)

umsetzt, in der $R_1$, $R_2$ und n die oben angegebenen Bedeutungen besitzen, welche man anschließend

— entweder mit einem Piperazin der allgemeinen Formel V

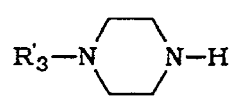

(V)

in der $R'_3$ eine Pyrimidinylgruppe, eine Phenylgruppe, die gegebenenfalls durch eine Alkylgruppe mit 1 bis 4 Kohlenstoffatomen oder eine Trifluormethylgruppe substituiert ist, oder eine Chinolylgruppe, die gegebenenfalls durch eine Nitrogruppe substituiert ist, bedeutet, zur Bildung einer Verbindung der allgemeinen Formel I, in der $R_3$ die gleichen Bedeutungen besitzt wie die Gruppe $R'_3$, umsetzt,

— oder mit 4-Benzyloxycarbonyl-piperazin der allgemeinen Formel VI

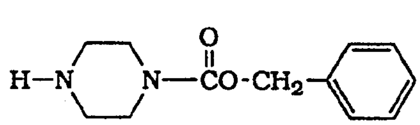

(VI)

umsetzt zur Bildung einer Verbindung der allgemeinen Formel VII

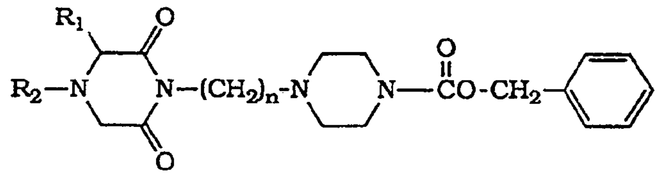

(VII)

in der $R_1$, $R_2$ und n die oben angegebenen Bedeutungen besitzen, welche man anschließend einer Acidolyse unterwirft zur Bildung eines Piperazinderivats der Formel VIII

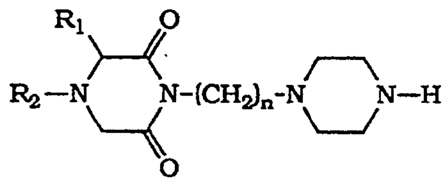

(VIII)

in der die Bedeutung von $R_1$, $R_2$ und n identisch ist mit der oben gegebenen, welches man mit einem Indolderivat der allgemeinen Formel IX

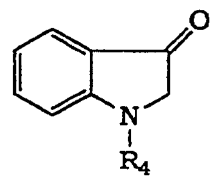

(IX)

in der $R_4$ eine Acylgruppe mit 1 bis 5 Kohlenstoffatomen bedeutet, umsetzt zur Bildung der Verbindungen der allgemeinen Formel I, in der $R_3$ eine Indolylgruppe darstellt, die am Stickstoffatom durch eine Acylgruppe mit 1 bis 5 Kohlenstoffatomen substituiert ist, und anschließend gewünschtenfalls die Verbindung der allgemeinen Formel I mit einer organischen oder anorganischen, pharmazeutisch annehmbaren Säure in ein Additionssalze überführt.

8. Pharmazeutische Zubereitung enthaltend als Wirkstoff eine Verbindung nach einem der Ansprüche 1 bis 6 in Kombination oder in Mischung mit einem inerten, nichttoxischen, pharmazeutisch annehmbaren Bindemittel oder Trägermaterial.

9. Pharmazeutische Zubereitung nach Anspruch 8, enthaltend den Wirkstoff in einer Dosis von 0,1 bis 100 mg.

10. Pharmazeutische Zubereitung nach den Ansprüchen 8 und 9 enthaltend als Wirkstoff mindestens eine Verbindung nach einem der Ansprüche 1 bis 6 zur Behandlung von Erkrankungen, welche die Verabreichung von anxiolytischen, antiaggressiven oder antipsychotischen Arzneimitteln notwendig machen.

## Claims

1. Compounds of the general formula I :

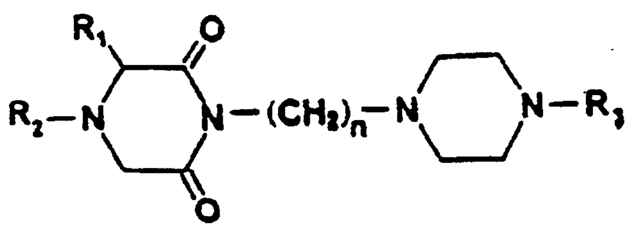

wherein

— either

— $R_1$ represents a hydrogen atom,

and
— $R_2$ represents a benzyl radical optionally substituted on the benzene ring by a halogen atom or by an alkoxy radical containing from 1 to 4 carbon atoms,
— or
— $R_1$ and $R_2$ together, and with the piperazine-2,6-dione radical to which they are attached, form a hexahydropyrazinoisoquinolinedione radical or a hexahydropyrazino-β-carbolinedione radical,
— $R_3$ represents a quinolyl radical optionally substituted by a phenyl radical or by a nitro radical, an indolyl radical substituted at the nitrogen atom by an acyl radical containing from 1 to 5 carbon atoms, or, with the proviso that $R_1$ does not at the same time represent a hydrogen atom, a pyrimidinyl radical, or a phenyl radical optionally substituted by an alkyl radical having from 1 to 4 carbon atoms or a trifluoromethyl radical, and
— $\underline{n}$ is an integer from 2 to 4,
and their addition salts formed with a pharmaceutically acceptable mineral or organic acid.

2. 4-(2-fluorobenzyl)-1-{3-[4-(6-nitro-2-quinolyl)-1-piperazinyl]-propyl}-piperazine-2,6-dione and its addition salts with a pharmaceutically acceptable mineral or organic acid.

3. 1-{3-[1-(1-acetyl-3-indolyl)-4-piperazinyl)-propyl}-4-(2-methoxybenzyl)-piperazine-2,6-dione and its addition salts with a pharmaceutically acceptable mineral or organic acid.

4. 2-{3-[4-(3-trifluoromethylphenyl)-1-piperazinyl]-propyl}-1,3,4,6,11,11a-hexahydro-2H-pyrazino[1,2-b]-isoquinoline-1,3-dione and its addition salts with a pharmaceutically acceptable mineral or organic acid.

5. 2-{4-[4-(2-pyrimidinyl)-1-piperazinyl]-butyl}-1,3,4,6,11,11a-hexahydro-2H-pyrazino[1,2-b]isoquinoline-1,3-dione and its addition salts with a pharmaceutically acceptable mineral or organic acid.

6. 2-{3-[4-(3-trifluoromethylphenyl)-1-piperazinyl]-propyl}-1,3,4,6,12,12a-hexahydro-2H-pyrazino[1,2-b]-β-carboline-1,3-dione and its addition salts with a pharmaceutically acceptable mineral or organic acid.

7. Process for the preparation of a compound of the general formula I, characterised in that:
— an imide of the general formula II :

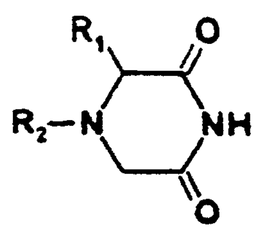

(II)

wherein $R_1$ and $R_2$ are as defined above for formula I, is reacted with a dihalogenated alkyl compound of the general formula III :

$$Br\text{-}(CH_2)_n\text{-}Cl \quad (III)$$

wherein $\underline{n}$ is an integer from 2 to 4, in the presence of an alkali metal hydride, to obtain the compound of the general formula IV :

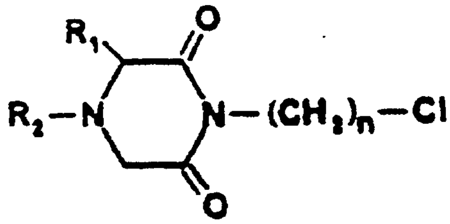

(IV)

wherein $R_1$, $R_2$ and $\underline{n}$ are as defined above, which compound is then condensed
— either with a piperazine of the general formula V :

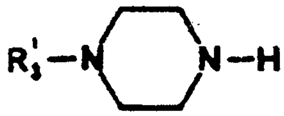

(V)

wherein $R'_3$ represents a pyrimidinyl radical, a phenyl radical optionally substituted by an alkyl radical having from 1 to 4 carbon atoms or a trifluoromethyl radical, or a quinolyl radical optionally substituted by a nitro group,
in order to obtain a compound of the general formula I wherein $R_3$ is as defined for $R'_3$,
— or with the 4-benzyloxycarbonylpiperazine of the general formula VI :

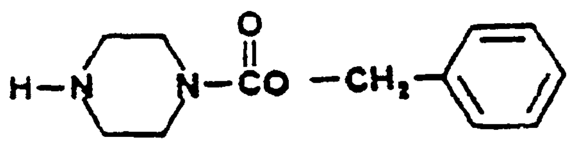

(VI)

to obtain a compound of the general formula VII :

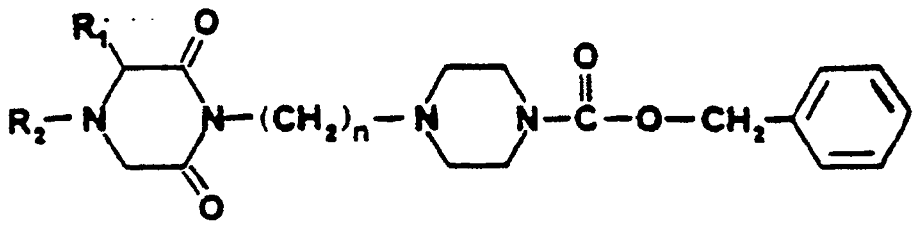

(VII)

wherein $R_1$, $R_2$ and n are as defined above,
which compound is then subjected to acidolysis to obtain a piperazine compound of the formula VIII,

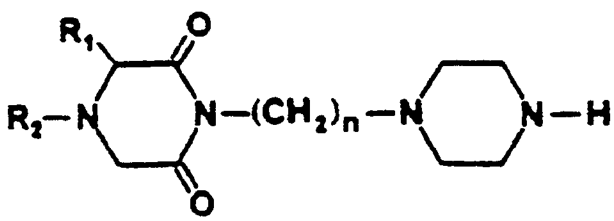

(VIII)

wherein $R_1$, $R_2$ and n are as defined above,
which compound is condensed with an indole compound of the general formula IX :

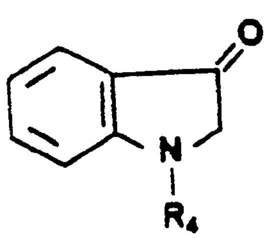

(IX)

wherein $R_4$ represents an acyl radical containing from 1 to 5 carbon atoms,
to form the compounds of the general formula I wherein $R_3$ represents an indolyl radical substituted at the nitrogen atom by an acyl radical containing from 1 to 5 carbon atoms,
and then, if desired,
the compounds of the general formula I are converted into an addition salt with a pharmaceutically acceptable organic or mineral acid.

8. Pharmaceutical composition containing as active in; dient a compound according to any one of claims 1 to 6 in conjunction or in admixture with a pharmaceutica acceptable non-toxic inert excipient or carrier.

9. Pharmaceutical composition according to claim 8 containing the active ingredient at a dose of from 0.1 to 100 mg.

10. Pharmaceutical composition according to claims 8 and 9 containing as active ingredient at least one compound according to claims 1 to 6 that can be used in the treatment of disorders necessitating anxiolytic, antiaggressive or antipsychotic medication.